# EUROPEAN PATENT APPLICATION

(11) **EP 3 704 989 A1**
(43) Date of publication of application: **09.09.2020**
(21) Application number: 18874936.0
(22) Date of filing: 01.11.2018
(51) Int. Cl.: A43D 1/02, A43B 17/00

(54) **INFORMATION PROCESSING DEVICE, INFORMATION PROCESSING SYSTEM, INSOLE PRODUCTION SYSTEM, INSOLE PRODUCTION METHOD, INFORMATION PROCESSING METHOD, AND PROGRAM**

(30) Priority: 02.11.2017 JP 2017212991
(71) Applicant: JSR Corporation, Tokyo 105-8640 (JP)
(72) Inventor: KOBE, Takashi, Tokyo 105-8640 (JP); KIYAMA, Satoshi, Tokyo 105-8640 (JP); KAWASE, Reiji, Tokyo 105-8640 (JP); HAYASHIDA, Taizo, Tokyo 105-8640 (JP)
(74) Representative: TBK
(86) International application number: PCT/JP2018/040747
(87) International publication number: WO 2019/088232

(57) **Abstract**

An information processing device (20) according to the invention includes a model data generator (205). The model data generator (205) generates model data to be used for a shaping device to shape an insole to be used by a subject, based on foot information generated based on image data of the subject and including outer shape data of a foot of the subject and data on bones of the foot of the subject.

## Description

### Field

The present invention relates to an information processing device, an information processing system, an insole production system, an insole production method, an information processing method, and a computer program. Background

Conventionally, therapeutic insoles are designed in the processes of obtaining, making, and correcting a mold of a foot by a prosthetist. However, it takes time and cost for the processes, and qualities vary between designers (prosthetists) with different experience levels. Therefore, in recent years, to reduce the time and cost required for designing an insole and stabilize the quality, the development of various techniques has been progressed.

For example, Patent Literature 1 discloses a technique that enables input of design parameters for determining a surface shape of an insole by defining the design parameters using the positions of bones of a foot as references and selecting types of the bones by a designer.

### Citation List

### Patent Literature

Patent Literature 1: International Publication No. WO2014/042094.

### Summary

### Technical Problem

In the foregoing conventional technique, however, since the positions of bones of a foot of an insole user are estimated from dimensions of the outer shape of the foot of the user, an insole with a surface shape suitable for the user has not been able to be designed depending on the accuracy of the estimation of the positions of the bones in some cases. It is noted that an insole is also referred to as a shoe insert, an inner sole, or the like. Hereinafter, a section described as an "insole" can be interpreted as a "shoe insert", an "inner sole", or the like.

The invention aims to provide an information processing device, a system, an information processing method, and a computer program that enable an insole suitable for a user to be easily designed.

### Solution to Problem

In order to solve the problem described above and to achieve the goal, in the present invention, the present invention discloses an information processing device comprising:
a model data generator that generates model data to be used for a shaping device to shape an insole to be used by a subject, based on foot information generated based on image data of the subject and including outer shape data of a foot of the subject and data on bones of the foot of the subject.

Further, the present invention discloses an information processing device comprising:
a foot information generator that generates, based on image data of a subject, foot information including outer shape data of a foot of the subject and data on bones of the foot of the subject; and
a model data generator that generates, based on the foot information, model data to be used for a shaping device to shape an insole to be used by the subject.

Further, the present invention discloses an information processing system comprising:
a foot information generator that generates, based on image data of a subject, foot information including outer shape data of a foot of the subject and data on bones of the foot of the subject;
a model data generator that generates, based on the foot information, model data to be used for a shaping device to shape an insole to be used by the subject;
a display device that displays the model data generated by the model data generator together with the foot information; and
a receiver that receives an operation of correcting the model data from a user who references the model data and the foot information displayed by the display device, wherein
the model data generator corrects the model data based on the operation received by the receiver.

Further, the present invention discloses an insole production system including at least an information processing device and a shaping device, comprising:
a foot information generator that generates, based on image data of a subject, foot information including outer shape data of a foot of the subject and data on bones of the foot of the subject;
a model data generator that generates, based on the foot information, model data to be used for the shaping device to shape an insole to be used by the subject; and
a shaping unit that shapes the insole based on the model data.

Further, the present invention discloses an insole production method comprising:
a foot information generation step of generating, based on image data of a subject, foot information including outer shape data of a foot of the subject and data on bones of the foot of the subject;
a model data generation step of generating, based on the foot information, model data to be used for a shaping device to shape an insole to be used by the subject; and
a shaping step of shaping the insole based on the model data.

Further, the present invention discloses an information processing method comprising:
a foot information generation step of generating, based on image data of a subject, foot information including outer shape data of a foot of the subject and data on bones of the foot of the subject; and
a model data generation step of generating, based on the foot information, model data to be used for a shaping device to shape an insole to be used by the subject.

Further, the present invention discloses a computer program that causes a computer to execute:
a foot information generation step of generating, based on image data of a subject, foot information including outer shape data of a foot of the subject and data on bones of the foot of the subject; and
a model data generation step of generating, based on the foot information, model data to be used for a shaping device to shape an insole to be used by the subject. Advantageous Effects of Invention

According to the invention, it is possible to easily design an insole suitable for a user.

### Brief Description of Drawings

FIG. 1 is a diagram illustrating an example of a schematic configuration of a system according to an embodiment.
FIG. 2 is a diagram illustrating an example of a hardware configuration of an information processing device.
FIG. 3 is a diagram illustrating an example of functions included in the information processing device.
FIG. 4 is a diagram illustrating an example of a screen on which image data of a foot is displayed.
FIG. 5 is a diagram illustrating an example of outer shape data of feet.
FIG. 6 is a diagram describing a database stored in a storage unit.
FIG. 7 is a (first) diagram describing model data.
FIG. 8 is a (second) diagram describing the model data.
FIG. 9 is a diagram describing a change in hardness of a boundary part.
FIG. 10 is a diagram illustrating an example of internal structures specified in hardness data.
FIG. 11 is a diagram illustrating a display example of the model data.
FIG. 12 is a diagram illustrating an example of a correction screen for the model data.
FIG. 13 is a flowchart illustrating an operation example of the information processing device according to the embodiment.
FIG. 14 is a diagram describing image data of a subject according to a modification.

### Description of Embodiment

Hereinafter, an embodiment of an information processing device, an information processing system, an insole production system, an insole production method, an information processing method, and a computer program according to the invention is described with reference to the accompanying drawings. The invention, however, is not limited to only the embodiment and can be variously modified without departing from the gist of the invention. An insole described in the following embodiment is also referred to as a shoe insert, an inner sole, or the like. In the following embodiment, a section described as an "insole" can be interpreted as a "shoe insert", an "inner sole", or the like.

### (Embodiment)

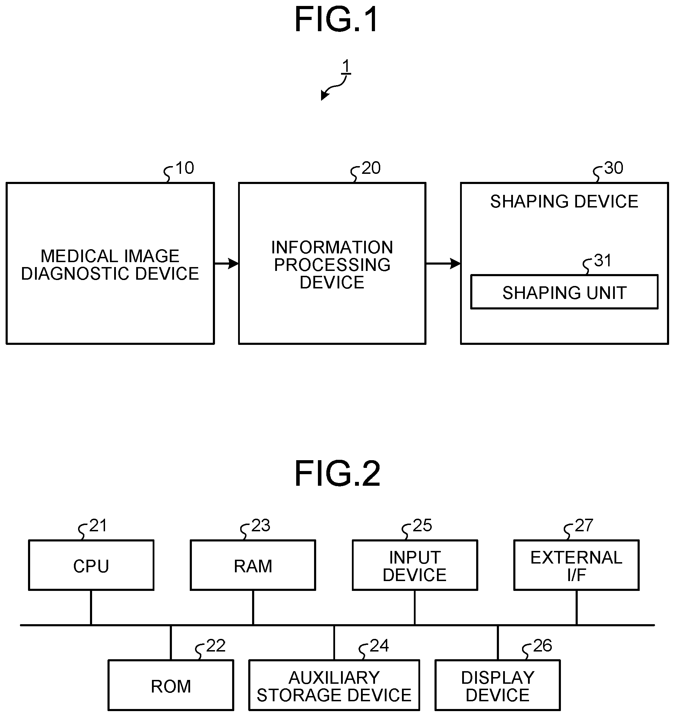

FIG. 1 is a diagram illustrating an example of a schematic configuration of a system 1 according to the embodiment. As illustrated in FIG. 1, the system 1 according to the embodiment includes a medical image diagnostic device 10, an information processing device 20, and a shaping device 30. The devices exemplified in FIG. 1 can communicate directly or indirectly with each other via a network that is a local area network (LAN), a wide area network (WAN), or the like.

The medical image diagnostic device 10 is a device that generates image data obtained by imaging a normally invisible part without damaging a human body. Specifically, the medical image diagnostic device 10 illustrated in FIG. 1 is a device that can generate three-dimensional image data (volume data) obtained by drawing at least the contour of a surface of an imaged part of the subject and a bone existing in the imaged part. For example, the medical image diagnostic device 10 is an X-ray computed tomography (CT) device, a magnetic resonance imaging (MRI) device, or the like. Imaging to be performed by the X-ray CT device that is an example of the medical image diagnostic device 10 is briefly described below.

The X-ray CT device performs imaging with a mounting device having a rotatable rotation frame holding an X-ray tube and an X-ray detector at positions where the X-ray tube and the X-ray detector are arranged opposite to each other. The X-ray tube emits an X-ray and an X-ray detector detects the X-ray that has passed through the subject. The X-ray CT device rotates the rotation frame while emitting an X-ray from the X-ray tube, thereby collecting projection data and reconstructing X-ray CT image data from the projection data. The X-ray CT image data is, for example, a tomographic image (two-dimensional X-ray CT image data) of a rotation plane (axial plane) between the X-ray tube and the X-ray detector.

In the X-ray detector, a plurality of detection element rows in which X-ray detection elements are arrayed in a channel direction are arrayed in a rotational axis direction of the rotation frame. For example, the X-ray CT device that includes the X-ray detector in which the 16 detection element rows are arrayed reconstructs a plurality (for example, 16) of tomographic images arranged in a body axis direction of the subject from projection data collected by causing the rotation frame to make one rotation. In addition, the X-ray CT device rotates the rotation frame and performs helical scan to move the subject or the mounting device, thereby, for example, reconstructing, as three-dimensional X-ray CT image data, 500 tomographic images of the entire heart.

In this case, the X-ray CT device as the medical image diagnostic device 10 illustrated in FIG. 1 is a device that can image the subject in a standing, sitting, or lying state. For example, the X-ray CT device images the subject sitting on a chair made of a material with high radiolucency and generates three-dimensional X-ray CT image data.

The MRI device can reconstruct an MRI image of an arbitrary one cross section or MRI images (volume data) of a plurality of arbitrary cross sections from MR signals collected by changing a gradient magnetic field for phase encoding, a gradient magnetic field for slice selection, and a gradient magnetic field for frequency encoding.

In the embodiment, the medical image diagnostic device 10 generates, as image data, three-dimensional X-ray CT image data obtained by capturing an image including a foot of the subject in a sitting state. Then, the medical image diagnostic device 10 transmits the generated image data to the information processing device 20.

Specifically, the medical image diagnostic device 10 converts the image data of the subject into Digital Imaging and Communications in Medicine (DICOM) data in a format conforming to the DICOM standard and transmits the DICOM data to the information processing device 20. The medical image diagnostic device 10 generates the DICOM data having the image data with additional information added thereto. The additional information includes a patient ID uniquely identifying the subject, patient information (name, gender, age, and the like), the type of inspection in which the image has been captured, an inspected part (imaged part), the state of the patient at the time of the imaging, and information on the image size. The information on the image size is used as information to be used to convert a length in an image space into a length in an actual space. The embodiment is applicable even in the case where the medical image diagnostic device 10 transmits, to the information processing device 20, three-dimensional X-ray CT image data obtained by capturing an image including the foot of the subject in a standing or lying state as the image data.

The information processing device 20 acquires, from the medical image diagnostic device 10, the image data of the subject (user) wearing an insole. Therefore, the information processing device 20 acquires, from the medical image diagnostic device 10, the three-dimensional X-ray CT image data obtained by capturing the image including the foot of the subject.

Then, the information processing device 20 uses various information generated from the image data of the subject to generate model data to be used for the shaping device 30 to shape an insole to be worn by the subject. Details of the foregoing are described later. Then, the information processing device 20 gives the generated model data to the shaping device 30.

The shaping device 30 includes a shaping unit 31 that shapes the insole to be worn by the subject based on the model data received from the information processing device 20. In the embodiment, the shaping unit 31 is composed of a hardware element group for providing a function of a three-dimensional printer (3D-printer).

The configuration of the shaping unit 31 that provides the function of the three-dimensional printer can be achieved by known various configurations. For example, the shaping unit 31 includes a nozzle for discharging a material heated and melted to a desired temperature and desired pressure, a moving mechanism for moving the nozzle in three-dimensional directions, a shaping stage on which pattern layers in desired shapes are formed of the material discharged from the nozzle, and a controller for controlling each of the units. The shaping unit 31 repeatedly stacks the pattern layers based on the model data, thereby shaping a three-dimensional structural body corresponding to the model data. One pattern layer is formed based on one tomographic image corresponding to the same position as the one pattern layer among a plurality of tomographic images constituting the model data.

Next, the information processing device 20 according to the embodiment is described. FIG. 2 is a diagram illustrating an example of a hardware configuration of the information processing device 20. As illustrated in FIG. 2, the information processing device 20 includes a central processing unit (CPU) 21, a read only memory (ROM) 22, a random access memory (RAM) 23, an auxiliary storage device 24, an input device 25, a display device 26, and an external I/F 27.

The CPU 21 executes a computer program to control an overall operation of the information processing device 20 and implement various functions included in the information processing device 20. The various functions included in the information processing device 20 are described later.

The ROM 22 is a nonvolatile memory and stores various data (information written at a stage of producing the information processing device 20) including a computer program for activating the information processing device 20. The RAM 23 is a volatile memory including a work region for the CPU 21. The auxiliary storage device 24 stores various data of the computer program to be executed by the CPU 21 and the like. The auxiliary storage device 24 is composed of, for example, a hard disk drive (HDD) or the like.

The input device 25 is a device to be used by a user (prosthetist) using the information processing device 20 to perform various operations. The input device 25 is composed of, for example, a mouse, a keyboard, a touch panel, or hardware keys.

The display device 26 displays various information. For example, the display device 26 displays the image data, the model data, graphical user interfaces (GUIs) for receiving various operations from the user, a medical image, and the like. The display device 26 is, for example, composed of a liquid crystal display, an organic electro luminescence (EL) display, or a cathode-ray tube display. For example, the input device 25 and the display device 26 may be unified and configured in the form of a touch panel or the like.

The external I/F 27 is an interface for connection to (communication with) external devices that are the medical image diagnostic device 10, the shaping device 30, and the like.

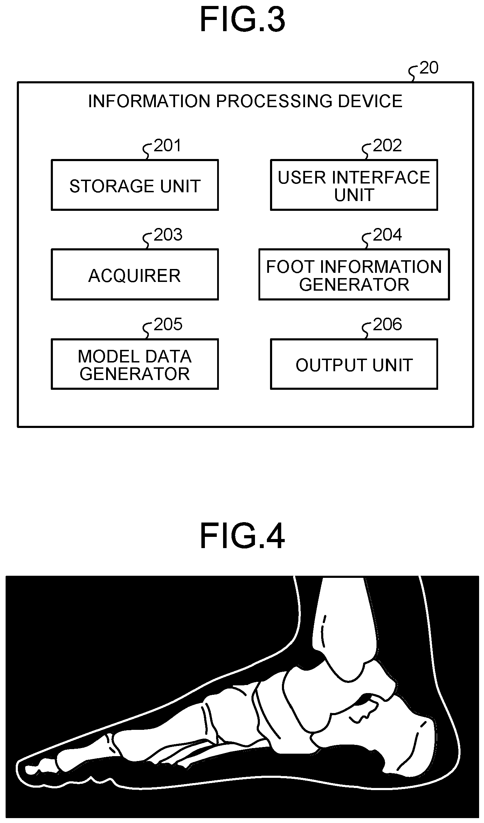

FIG. 3 is a diagram illustrating an example of the functions included in the information processing device 20. Although only functions related to the embodiment are illustrated in the example of FIG. 3, the functions included in the information processing device 20 are not limited to them. As illustrated in FIG. 3, the information processing device 20 includes a storage unit 201, a user interface unit 202, an acquirer 203, a foot information generator 204, a model data generator 205, and an output unit 206.

The storage unit 201 is, for example, achieved by the auxiliary storage device 24 (for example, HDD) illustrated in FIG. 2. The storage unit 201 stores the image data (DICOM data) acquired from the medical image diagnostic device 10, a database to be referenced in the generation of the model data, and the like.

The user interface unit 202 includes a function of receiving input of the user and a function of displaying various information. For example, when the user interface unit 202 receives, from the user, an operation of calling the image data of the subject to generate an insole, the user interface unit 202 reads the image data specified by the operation from the storage unit 201 and performs control to display the image data on the display device 26. FIG. 4 is a diagram illustrating an example of a screen on which the image data of the foot is displayed. FIG. 4 illustrates three-dimensional X-ray CT image data displayed on the screen of the display device 26. As illustrated in FIG. 4, a surface (contour) of the foot of the subject and various types of bones constituting foot bones are drawn in the three-dimensional X-ray CT image data.

To display the three-dimensional image data on the two-dimensional screen, various rendering processes are performed on the three-dimensional image data. As the rendering processes, a volume rendering process, a surface rendering process, and multi-planar reconstruction (MPR) are exemplified. In the volume rendering process, two-dimensional image data in which three-dimensional information of the volume data is reflected can be generated. In addition, in the surface rendering process, arbitrary surface information can be extracted from the volume data, and two-dimensional image data can be reconstructed. Furthermore, in the MPR, MPR image data of an arbitrary cross section can be reconstructed from the volume data. In the embodiment, the model data generator 205 described later performs the various rendering processes on the three-dimensional image data. An image illustrated in FIG. 4 indicates an image obtained by performing the surface rendering process on the three-dimensional X-ray CT image data.

Returning to FIG. 3, the acquirer 203 acquires various data including the image data of the subject and necessary to design an insole via the external I/F 27. For example, the acquirer 203 causes the image data, received from the medical image diagnostic device 10, of the subject to be stored in the storage unit 201. Then, the foot information generator 204 acquires the image data of the subject from the storage unit 201.

Then, the foot information generator 204 generates, based on the image data of the subject, foot information including outer shape data of the foot of the subject and data on bones of the foot of the subject. For example, the foot information generator 204 performs known image processing such as edge detection process on the three-dimensional X-ray CT image data to extract the contour of the foot. Therefore, the foot information generator 204 acquires the outer shape data three-dimensionally indicating a surface shape of the foot of the subject.

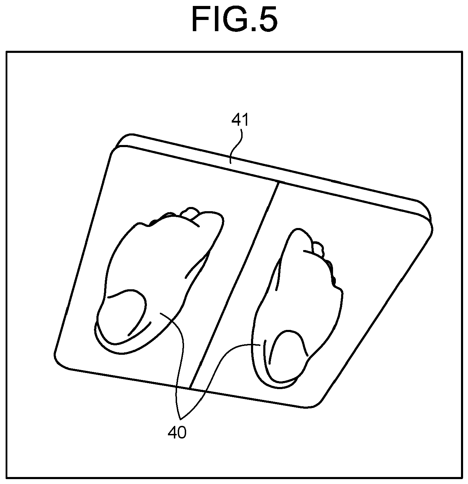

FIG. 5 is a diagram illustrating an example of outer shape data of feet. FIG. 5 illustrates image data obtained by adjusting the position of a point of view and a line-of-sight direction and performing the surface rendering process so that the three-dimensional outer shape data is viewed from the soles of the feet. The image data illustrated in FIG. 5 includes outer shape data 40 of the feet and data 41 indicating an outer shape of a measurement stage on which the subject has rested.

In addition, the foot information generator 204 references a database in which types of bones, shapes of the bones, and position information of the bones are associated with each other and stored, and generates data related to the bones of the foot of the subject and including information on the types of the bones, the shapes of the bones, and the positions of the bones. First, the foot information generator 204 performs a known segmentation process on the three-dimensional X-ray CT image data and extracts a three-dimensional region (three-dimensional bone region) corresponding to the bones. For example, the foot information generator 204 uses a predetermined threshold to binarize and extract voxels corresponding to CT values of the bones, thereby extracting the three-dimensional bone region. Then, the foot information generator 204 references the database and identifies the shapes, types, and positions of the plurality of bones included in the extracted three-dimensional bone region.

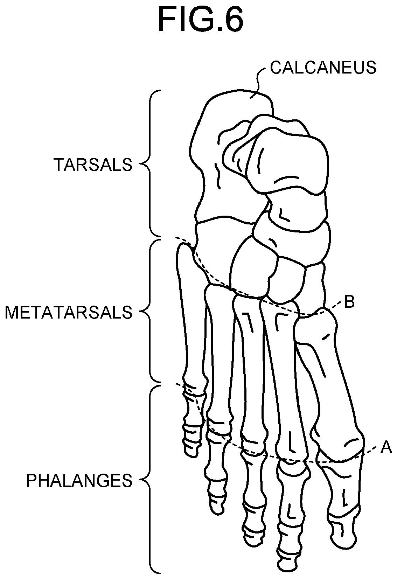

In the embodiment, the foregoing database is stored in the storage unit 201. FIG. 6 is a diagram describing the database stored in the storage unit 201. FIG. 6 indicates a template for detecting each of bones constituting foot bones via pattern matching. In FIG. 6, a top view of the template indicating standard bones of a right foot is illustrated, a direction from the top to the bottom is a direction from a heel to tips of toes, and a direction from the right to the left is a direction from a bit toe to a little toe.

The template illustrated in FIG. 6 indicates shapes of the various bones constituting the foot bones. In addition, anatomical names indicating the types of the bones are associated with the bones of the template. For example, the database stores the fact that bones illustrated on the lower side with respect to a curve A in FIG. 6 are phalanges, bones illustrated between the curve A and a curve B in FIG. 6 are metatarsals, and bones illustrated on the upper side with respect to the curve B in FIG. 6 are tarsals. The curve A extends through metatarsophalangeal joints and extends through ends (heads) of the five metatarsals on the toe side. In addition, the curve B extends through Lisfranc joints and extends through ends (bases) of the five metatarsals on the heel side.

In addition, the database stores the fact that the names of the five metatarsals are the first metatarsal, the second metatarsal, the third metatarsal, the fourth metatarsal, and the fifth metatarsal in the order from the right side in FIG. 6. Furthermore, the database is composed of a talus, a navicular, a cuboid, a calcaneus, and a cuneiform that constitute the tarsals, and stores relations of the arrangement of these bones. For example, the database stores the fact that the calcaneus constituting the tarsal exists at a medial posterior surface of the foot.

The foot information generator 204 performs pattern matching on the template illustrated in FIG. 6 and the three-dimensional bone region extracted by the segmentation process from the three-dimensional X-ray CT image data and generates the data including the information on the types of the bones, the shapes of the bones, and the positions of the bones and related to the bones of the foot. As a method for the pattern matching, template matching for determining whether it matches predetermined template data, and multivariable analysis for making a determination based on characteristic amounts related to the types of the bones, the shapes of the bones, and the positions of the bones are exemplified. In addition, it is preferable that the data on the bones of the foot include position information of the metatarsals and the calcaneus.

The database may store templates indicating foot bones in standard shapes for gender and age, like a template of "males, 8-year-old to 10-year-old" and a template of "females, 20-year-old to 30-year-old". In this case, the foot information generator 204 acquires the gender and age of the subject from the additional information of the DICOM data, reads a template corresponding to the acquired gender and the acquired age from the database, and generates the data on the bones of the foot.

Returning to FIG. 3, the model data generator 205 generates, based on the foot information, model data to be used for the shaping device 30 to shape an insole to be used by the subject. First, before the model data generator 205 starts a process of generating the model data, the user uses the input device 25 to input information (shoe shape information) on a shoe of the subject and the shape of an insole attached to the shoe in advance. As the shoe shape information, dimension information of the shoe, shape information of tips of toes, or dimension information or shape information of the insole attached in advance can be used. The dimension information of the shoe is, for example, dimension information of a length, a width, and the like of the shoe. The shape information of the tips of the toes is, for example, shape pattern information of a pointed toe shape, a round toe shape, or square toe shape. In addition, the dimension information of the insole attached in advance is, for example, dimension information of the insole corresponding to the foot length and the foot width. The foot length is a length from the most protruding part of the heel to an end of the longest part among the toes of the foot, while the foot width is a width from a root part of the big toe to a root part of the little toe. Furthermore, the shape information of the insole attached in advance is, for example, planar shape data or three-dimensional shape data of the insole attached in advance.

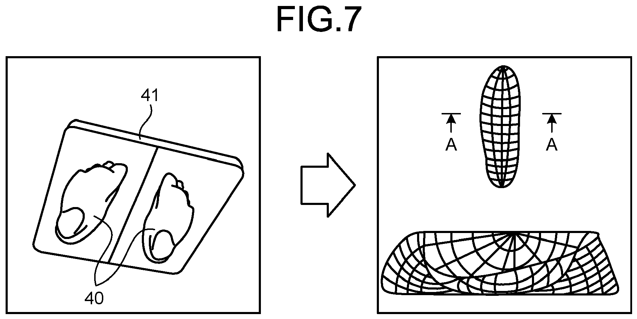

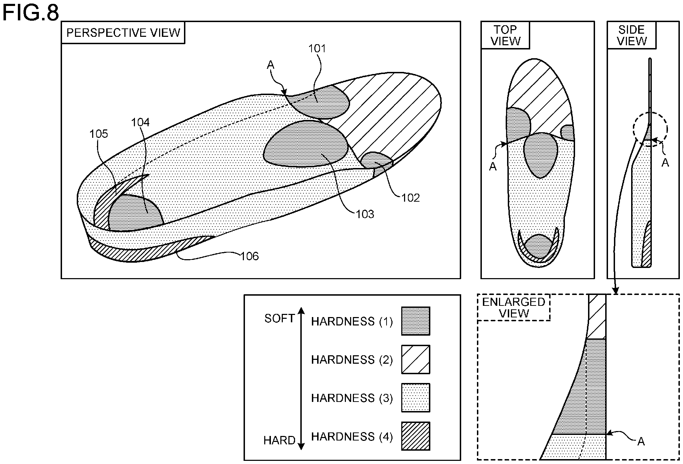

The acquirer 203 causes the shoe shape information received by the user interface unit 202 to be stored in the storage unit 201. Then, the model data generator 205 acquires the shoe shape information from the storage unit 201. Then, the model data generator 205 generates outer shape data indicating the outer shape of an insole based on the foot information. In this example, the model data generator 205 generates the outer shape data indicating the outer shape of the insole based on the foot information and the shoe shape information. In addition, the model data generator 205 generates, based on the foot information, hardness data indicating hardness at each of positions on the insole whose outer shape has been defined based on the outer shape data. Hereinafter, for convenience, a surface of the insole that will be in contact with a shoe is referred to as a back surface, and a surface of the insole that will be in contact with the sole of the foot is referred to as a front surface. FIGS. 7 and 8 are diagrams describing the model data. FIG. 7 illustrates an example of the outer shape data, and FIG. 8 illustrates the model data including the outer shape data and the hardness data. In addition, a curve A illustrated in FIG. 8 is a curve drawn based on the position of a middle toe described using FIG. 6.

For example, when the "shoe shape information: the shape information of the insole attached in advance" is received, the model data generator 205 sets the shape of the back surface based on the shape information of the insole attached placed in advance. Then, the model data generator 205 references the outer shape data of the foot and sets a height from the back surface for each of the positions so that the front surface is in a shape based on the sole of the subject. Therefore, the model data generator 205 converts the outer shape data, illustrated in a left graphic of FIG. 7, of the foot into outer shape data of an insole illustrated in a right graphic of FIG. 7. The outer shape data of the insole is, for example, data including three-dimensional position data of the back surface of the insole and data indicating a height from the back surface to the front surface at each of the positions or is height data associated with position data or data indicating the outer shape of the insole. An upper left graphic of FIG. 7 is a graphic of the outer shape data of the insole viewed from above, and a lower left graphic of FIG. 7 illustrates a cross section obtained by cutting the outer shape data of the insole at a cross section extending through left and right ends of the curve A illustrated in FIG. 6. The data format of the outer shape data is, for example, stereolithography (STL) data. As illustrated in FIG. 8, the model data generator 205 generates, as initial settings, outer shape data of a shape including side surfaces of the foot and the back surface of the foot on the heel side with respect to the curve A based on the outer shape data of the foot. In addition, as illustrated in FIG. 8, the model data generator 205 generates, as initial settings, outer shape data of a fixed thickness on the tiptoe side with respect to the curve A, regardless of the shape indicated by the outer shape data of the foot.

Then, the model data generator 205 sets hardness at each of the positions on the insole based on the data on the bones of the foot. Basic information for defining hardness at each of the positions on the insole described below is, for example, based on information collected from a prosthetist with a high experience level and is set in the model data generator 205 in advance.

For example, as illustrated in FIG. 8, the model data generator 205 sets four types of hardness, hardness (1), hardness (2), hardness (3), and hardness (4), while the hardness increases in the order of the hardness (1), the hardness (2), the hardness (3), and the hardness (4). In the example illustrated in FIG. 8, the model data generator 205 sets, to the hardness (1), hardness of a region 101 of the hallux ball defined by the position of the head of the first metatarsal, a region 102 of the little toe ball defined by the position of the head of the fifth metatarsal, a region 103 of a central part corresponding to the second to the fourth metatarsals, and a region 104 of the heel defined by the position of the calcaneus. In addition, in the example illustrated in FIG. 8, the model data generator 205 sets, to the hardness (2), hardness of a region that exists on the tiptoe side with respect to the curve A and excludes the region 101 and the region 102.

In addition, in the example illustrated in FIG. 8, the model data generator 205 sets, to the hardness (4), hardness of a region 105 that will be in contact with a back part of the heel and a region 106 that is arranged opposite to the region 105 on the back surface side and will be in contact with a shoe. In addition, in the example illustrated in FIG. 8, the model data generator 205 sets, to the hardness (3), hardness of a region that exists on the heel side with respect to the curve A and excludes the region 103, the region 104, the region 105, and the region 106. In the foregoing example, hardness that varies in position in a height direction is set for the heel.

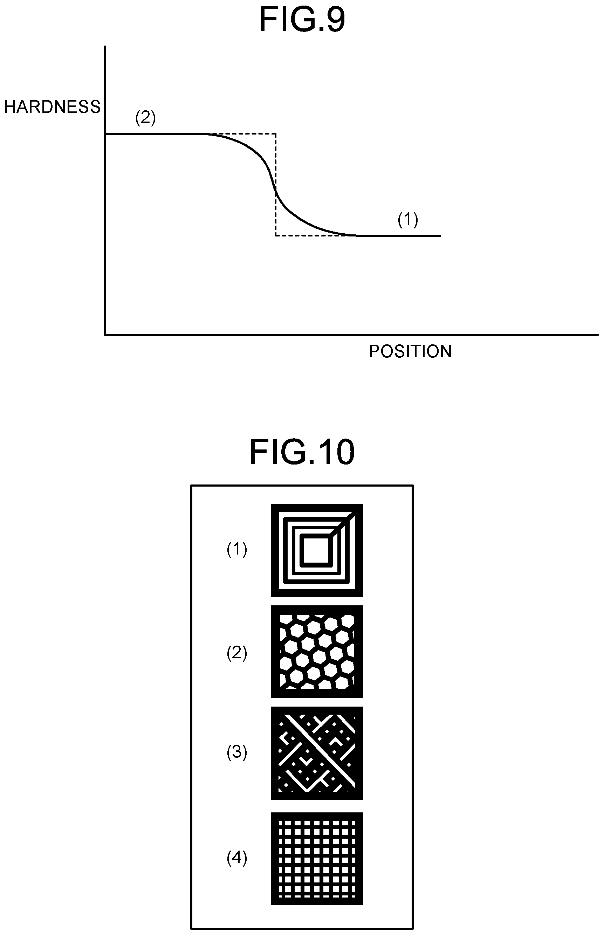

Furthermore, the model data generator 205 generates hardness data indicating that hardness gradually changes at boundary parts where hardness changes. As a method for generating the hardness data indicating that the hardness gradually changes, a metaball algorithm or a morphing algorithm can be exemplified. FIG. 9 is a diagram describing a change in hardness at a boundary part. For example, as indicated by a dotted line in FIG. 9, if the hardness rapidly changes from the hardness (2) to the hardness (1) at a boundary part in the region 101, there is a possibility that a comfortable wearing feeling for the user cannot be obtained. Therefore, as indicated by a solid line in FIG. 9, the model data generator 205 generates the hardness data indicating that the hardness gradually changes from the hardness (2) to the hardness (1) at the boundary part in the region 101.

In addition, the model data generator 205 generates hardness data specifying an internal structure for each of different levels of hardness. FIG. 10 is a diagram illustrating an example of the internal structures specified in the hardness data. FIG. 10 illustrates internal structures defining resin densities corresponding to the hardness (1) to the hardness (4). Internal structure data in which the internal structures are associated with the hardness is stored in the storage unit 201, and the model data generator 205 references the storage unit 201 and designs an internal structure at each position in the model data.

In this manner, the model data generator 205 generates the model data including the hardness data associated with the outer shape data of the insole and the position data of the insole. The model data generator 205 generates the model data based on the foot information. In the embodiment, the model data generator 205 generates the model data based on the shoe shape information as well as the foot information. When the model data generator 205 generates the model data, the user interface unit 202 controls the display device 26 to cause the display device 26 to display the model data. In the embodiment, the user interface unit 202 controls the display device 26 to cause the display device 26 to display the model data together with the foot information.

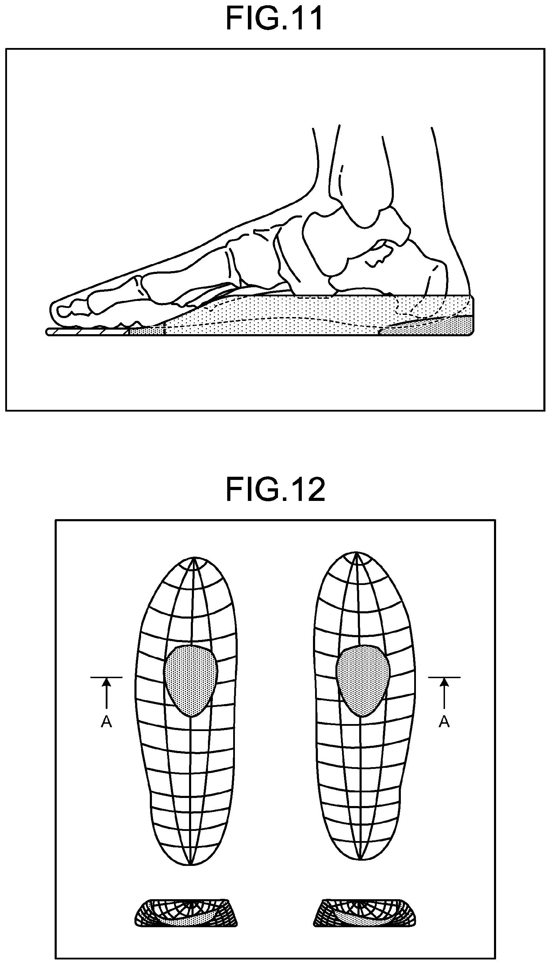

For example, the model data generator 205 performs the volume rendering process to generate image data for display based on the position data included in the model data in accordance with an instruction of the user interface unit 202 in a state in which the model data, the outer shape data of the foot, and the data on the bones of the foot are arranged in a three-dimensional space. FIG. 11 is a diagram illustrating a display example of the model data. Therefore, as illustrated in FIG. 11, the display device 26 displays the image data in which the model data generated by the model data generator 205, and the outer shape data of the foot and the data on the bones of the foot, are superimposed. FIG. 11 exemplifies the model data viewed from the inner side, the outer shape data of the foot, and the data on the bones of the foot. The embodiment may be applied to the case where a display request is received from the user and the model data is displayed. In addition, display and non-display of the foot information are set by the user so that the user can switch between the display and the non-display.

The user adjusts the position of a point of view and a line-of-sight direction and confirms the model data from various directions. The user references the model data displayed by the display device 26 and the foot information displayed by the display device 26 and uses the input device 25 to perform an operation of correcting the model data. Upon receiving the operation of correcting the model data from the user, the user interface unit 202 notifies the received operation to the model data generator 205. The model data generator 205 corrects the model data based on the operation received by the user interface unit 202. FIG. 12 is a diagram illustrating an example of a correction screen for the model data.

For example, when the data on the bones of the foot is referenced and it is determined that a lateral arch of the subject is not normal, the user specifies the central part (region 103 illustrated in FIG. 8) corresponding to the second to the fourth metatarsals and performs an operation of correcting the height of the central part so that the lateral arch can be corrected. FIG. 12 illustrates a screen on which central parts are specified after the non-display of the foot information is set. The model data generator 205 corrects the height of the central part based on the operation by the user. For example, in the embodiment, the height data can be corrected by a sculpting process executed on the STL data. Upper graphics of FIG. 12 are graphics when outer shape data of insoles is viewed from above, like the upper right graphic of FIG. 7. Lower graphics of FIG. 12 illustrate cross sections obtained by cutting the outer shape data of the insoles at the cross section extending through the left and right ends of the curve A illustrated in FIG. 6, like the lower left graphic of FIG. 7.

The user can operate the input device 25 to perform correction on the hardness data and correction on the position data in the hardness data associated with the position data, as well as the correction of the outer shape data. The correction of the hardness data is, for example, a change in an internal structure of the region 101. In addition, the correction of the position data in the hardness data associated with the position data is, for example, the adjustment of the position of the region 101.

Returning to FIG. 3, the output unit 206 outputs the model data generated by the model data generator 205 to the shaping device 30. Alternatively, the output unit 206 outputs, to the shaping device 30, the model data corrected by the model data generator 205 based on an instruction operation by the user. After receiving, from the user, a request to output the model data generated by the model data generator 205 or the model data corrected by the model data generator 205, the output unit 206 performs a process of the outputting to the shaping device 30 via the external I/F 27. The shaping unit 31 of the shaping device 30 uses the model data received from the information processing device 20 to shape the insole. The shape of the insole shaped by the shaping device 30 is corrected by the user when necessary.

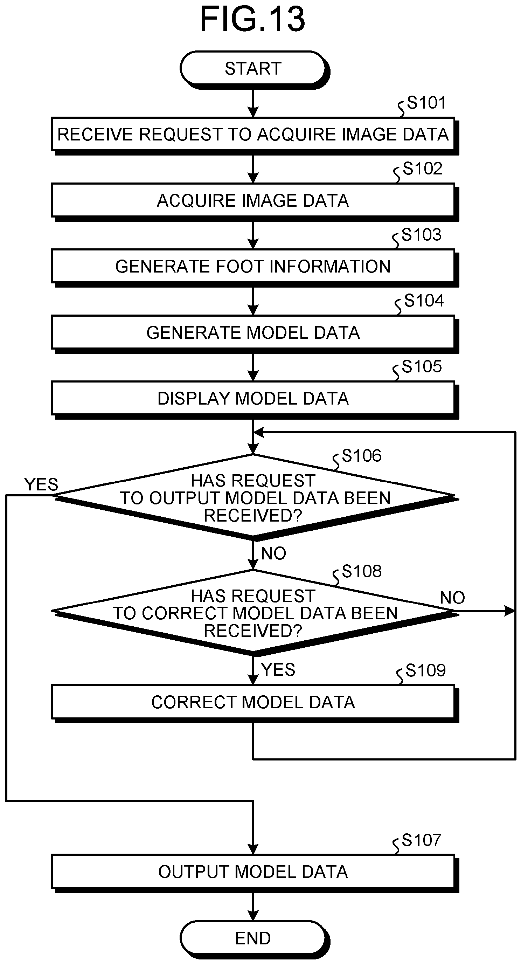

FIG. 13 is a flowchart illustrating an operation example of the information processing device 20 according to the embodiment. Since specific details of steps are described above, a detailed description thereof is omitted as appropriate.

As illustrated in FIG. 13, when the user interface unit 202 receives a request to acquire the image data (step S101), the acquirer 203 acquires the image data (step S102).

Then, the foot information generator 204 references the database and generates the foot information from the image data (step S103), and the model data generator 205 generates the model data based on the foot information (step S104). Then, the display device 26 displays the model data under control by the user interface unit 202 (step S105). At step S105, the display device 26 displays the foot information together with the model data.

Next, the user interface unit 202 determines whether the user interface unit 202 has received a request to output the model data (step S106). When the user interface unit 202 has received the request to output the model data (Yes at step S106), the output unit 206 outputs the model data to the shaping device 30 (step S107) and terminates a process.

On the other hand, when the user interface unit 202 has not received the request to output the model data (No at step S106), the user interface unit 202 determines whether the user interface unit 202 has received a request to correct the model data (step S108). When the user interface unit 202 has received the request to correct the model data (Yes at step S108), the model data generator 205 corrects the model data (step S109).

On the other hand, when the user interface unit 202 has not received the request to correct the model data (No at step S108) or after step S109, the user interface unit 202 causes the process to return to step S106 and determines whether the user interface unit 202 has received the request to output the model data. After step S109, when the user interface unit 202 has received the request to output the model data at step S106 (Yes at step S106), the output unit 206 outputs the model data after the correction to the shaping device 30 (step S107) and terminates the process.

As described above, the information processing device 20 according to the embodiment acquires, based on the image data of the subject, the foot information including the outer shape data of the foot of the subject and the data on the bones of the foot and generates the model data of the insole based on the foot information. Specifically, in the embodiment, accurate dimensions of the outer shape of the foot and accurate position information of the various bones constituting the foot bones can be generated based on the X-ray CT image data of the subject. As a result, in the embodiment, the model data of the surface shape suitable for the user (subject) can be easily designed.

In addition, in the embodiment, the model data including the hardness data in which the hardness has been set for each of the positions is generated based on the foot information. As a result, in the embodiment, it is possible to easily design the model data in which appropriate hardness has been set based on positions of the foot of the user.

In addition, in the embodiment, the user references the screen on which the model data and the foot information are superimposed, and can correct the model data. For example, the user references the outer shape data of the foot and the data on the bones of the foot and can correct the model data while recognizing a subject's symptom such as hallux valgus. As a result, in the embodiment, it is possible to easily design the model data to be used to care for the symptom of the foot of the user.

Although the embodiment of the invention is described above, the invention is not limited to the foregoing embodiment and can be embodied by modifying the constituent elements without departing from the gist thereof at an implementation stage. In addition, various inventions can be made by properly combining a plurality of constituent elements disclosed in the foregoing embodiment. For example, some constituent elements may be removed from all the constituent elements described in the embodiment.

### (Modifications)

Modifications are described below.

### (1) First Modification

Although the foregoing embodiment describes the case where the data is passed between the devices by the transmission and the reception via the network, the invention may be applied to the case where the data is passed via a storage medium that is MicroSD (registered trademark), a digital versatile disc (DVD), a BlueRay Disc (registered trademark), or the like. In addition, although the foregoing embodiment describes the case where the image data is acquired from the medical image diagnostic device 10, the invention may be applied to the case where image data is acquired from a medical image storage device storing the image data obtained by imaging by various medical image diagnostic devices.

### (2) Second Modification

Although the foregoing embodiment describes the case where the image data of the subject in the sitting state is obtained by the imaging, the invention may be applied to image data that includes the foot of the subject in a standing or lying state and is obtained by the imaging. Since the insoles are deformed due to loads on the feet of the user when the user wears the insoles, it is preferable that image data of the sitting state or the standing state be used as image data for the design of the insoles.

The state of the subject who is the user when the image data of the subject is obtained by the imaging may be a state other than the foregoing sitting state, the foregoing standing state, and the foregoing lying state. For example, in normal walking, it can be regarded that average loads are applied to both feet. Therefore, when a main behavior of the user wearing the insoles is normal walking, image data obtained by the imaging in the sitting or standing state in which loads are applied to both feet is used.

On the other hand, when the main behavior of the user wearing the insoles is, for example, running, a load is alternately applied to the right foot and the left foot. Therefore, to design insoles for running, image data obtained by the imaging with one leg standing may be used. For example, the model data generator 205 generates foot information of the right foot to which the entire load is applied, from image data obtained by imaging the right foot in a state in which the user stands on the right leg. In addition, the model data generator 205 generates foot information of the left foot to which the entire load is applied, from image data obtained by imaging the left foot in a state in which the user stands on the left leg. Since the model data generator 205 generates model data using the foot information of the left and right feet, insoles suitable for a behavior state (usage purpose) of the user can be designed.

In addition, the invention may be applied to the case where image data of the subject that has been obtained by the imaging in a plurality of types of states is used. For example, the foot information generator 204 acquires foot information from each of a plurality of types of subject's image data in which the ratios of loads applied to the left foot to loads applied to the right foot are different. Then, the foot information generator 204 uses a plurality of types of the foot information to generate average foot information indicating average foot states of the user, and the model data generator 205 generates model data based on the average foot information. Alternatively, the foot information generator 204 acquires one or multiple foot information items selected based on a main behavior of the user wearing insoles, and the model data generator 205 generates model data based on average foot information acquired from the selected one or multiple foot information items. By using a plurality of types of subject's image data from which foot information is acquired in the foregoing manner, the probability that insoles suitable for the user (subject) can be designed can be increased.

### (3) Third Modification

Although the foregoing embodiment describes the case where the generation of the foot information (outer shape data of the foot and data on the bones of the foot) is performed by the image analysis process by the foot information generator 204, the invention is not limited to this. The invention may be applied to the case where the foot information is generated by inputting the outer shape of the foot and outer shapes, types, and positions of the bones constituting the foot bones by a user (doctor or the like) who has referenced the image data of the subject. In addition, although the foregoing embodiment describes the case where the information processing device 20 analyzes the image data and generates the foot information, the invention is not limited to this. The invention may be applied to the case where the information processing device 20 acquires foot information generated from the image data by analysis by another device. Specifically, the invention may be applied to the case where the information processing device 20 including the model data generator 205 receives the foot information from an external device for performing the same processes as the foot information generator 204 and generates the model data.

### (4) Fourth Modification

Although the foregoing embodiment describes the case where the foot information generator 204 acquires the foot bone data including the position information of the metatarsals and the calcaneus, the invention is not limited to this. In the invention, it is sufficient if the position information of the bones in the foot bone data includes position information of at least one type of bone selected from the metatarsals and the calcaneus. Specifically, the foot information generator 204 may generate data including position information of either the metatarsals or the calcaneus and related to the bones of the foot. In the case where the foot information generator 204 does not generate the position information of the calcaneus from the image data of the subject, for example, the foot information generator 204 may estimate the position information of the metatarsals from the position information of the calcaneus and the outer shape data of the foot or acquire the position information of the metatarsals via input of the user. In addition, in the case where the foot information generator 204 does not generate position information of the metatarsals from the image data of the subject, for example, the foot information generator 204 may estimate the position information of the calcaneus from the position information of the metatarsals and the outer shape data of the foot or may acquire the position information of the calcaneus via input of the user. It is possible for the user to arbitrarily change whether the bone position information of the metatarsals and the calcaneus is acquired by the image analysis or the position information of either the metatarsals or the calcaneus is acquired by the image analysis.

### (5) Fifth Modification

Although the foregoing embodiment describes the case where the outer shape data (height data associated with the position data) of the insole and the hardness data associated with the position data are included as the model data, the invention is not limited to this. The invention may be applied to the case where the model data includes either the outer shape data of the insole or the hardness data. In this case, the other data that has not been selected is, for example, set by the user.

### (6) Sixth Modification

Although the foregoing embodiment describes the case where the foot information is superimposed on the model data, the invention may be also applied to the case where an abnormal part in the foot information is displayed so that the abnormal part can be identified. The abnormal part is specified by a doctor. Alternatively, the foot information generator 204 may use the foregoing template of the bones to detect an abnormal part of a bone. In addition, an outer shape template indicating average outer shapes of feet may be stored in the storage unit 201, and the foot information generator 204 may use the outer shape template to detect an externally abnormal part. By displaying the part so that the part can be identified, the correction process by the user can be supported.

### (7) Seventh Modification

Although the foregoing embodiment describes the case where the database to be used by the foot information generator 204 to generate the data on the bones of the foot is stored in the storage unit 201, the invention may be applied to the case where the database is stored in an external device that can communicate with the information processing device 20.

### (8) Eighth Modification

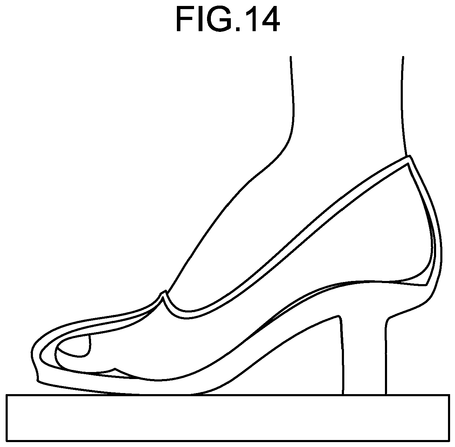

Although the foregoing embodiment describes the case where the shoe shape information is received from the user to define the shape of the back surface of the insole as the embodiment, receiving the shoe shape information is not mandatory. For example, the model data generator 205 may generate model data indicating that back surfaces of insoles are flat as initial settings. Alternatively, in the invention, the shoe shape information may be acquired from the image data of the subject. FIG. 14 is a diagram describing the image data of the subject according to the modification.

For example, as illustrated in FIG. 14, the image data of the subject may be data obtained by the imaging in a state in which the subject wears a shoe. FIG. 14 illustrates the case where the X-ray CT imaging is performed in a state in which the standing or sitting subject wears a high heel. The shoe illustrated in FIG. 14 is not a shoe actually used by the subject and is a shoe prepared for design of an insole. The shoe that has radiolucency not affecting drawing of the foot surface and the foot bones and is made of a material having radiolucency enabling the drawing of the contour of the shoe is used. By analyzing the image data, model data defining the shape of the back surface of the insole can be generated. As a result, a time period for performing the correction process based on the shape of the shoe can be reduced. In addition, the foot information generator 204 can acquire outer shape data close to the shape of the foot when the insole is worn.

### (9) Ninth Modification

Although the system 1 according to the foregoing embodiment serves as a production system for performing the insole production method and includes the medical image diagnostic device 10, the information processing device 20, and the shaping device 30, it is sufficient if the system 1 according to the embodiment serves as an insole production system and includes at least the foregoing information processing device 20 and the shaping device 30 for shaping an insole. In addition, for example, at least one of the functions included in the foregoing information processing device 20 may be included in the shaping device 30. For example, the system to which the invention is applied may include at least the foregoing acquirer 203, the foregoing model data generator 205, and the foregoing shaping unit 31, and these constituent elements may be optionally included in the information processing device 20 or the shaping device 30. For example, the foregoing model data generator 205 may be included in the shaping device 30.

### (10) Tenth Modification

The embodiment describes the case where, in the system 1 according to the foregoing embodiment, the information processing device 20 as the information processing system performs the series of processes, "(a) the generation of the foot information, (b) the generation of the model data, (c) the display of the model data and the foot information, (d) the reception of the operation of correcting the model data, and (e) the correction of the model data based on the operation". The series of processes, however, may be performed by different devices. For example, the processes (a) and (b) may be performed in a cloud environment, (c) and (d) may be performed by a terminal device such as a personal computer installed in a prosthesis maker, and the process (e) may be performed also in the cloud environment.

The foregoing embodiment can be arbitrarily combined with the foregoing modifications, and the foregoing modifications can be arbitrarily combined with each other.

In addition, the computer program to be executed by the information processing device 20 according to the foregoing embodiment may be configured so that the computer program is recorded in an installable format or an executable format in a computer-readable recording medium such as a CD-ROM, a flexible disk (FD), a CD-R, a DVD, or a universal serial bus (USB) and provided or is provided and distributed via a network. In addition, various computer programs may be configured so that the computer programs are stored in a nonvolatile storage medium such as, for example, a ROM in advance and provided.

### Reference Signs List

- 1: System
- 10: Medical image diagnostic device
- 20: Information processing device
- 30: Shaping device
- 31: Shaping unit
- 201: Storage unit
- 202: User interface unit
- 203: Acquirer
- 204: Generator
- 205: Output unit

## Claims

1. An information processing device comprising:
a model data generator that generates model data to be used for a shaping device to shape an insole to be used by a subject, based on foot information generated based on image data of the subject and including outer shape data of a foot of the subject and data on bones of the foot of the subject.

2. An information processing device comprising:
a foot information generator that generates, based on image data of a subject, foot information including outer shape data of a foot of the subject and data on bones of the foot of the subject; and
a model data generator that generates, based on the foot information, model data to be used for a shaping device to shape an insole to be used by the subject.

3. The information processing device according to claim 1 or 2, wherein
the model data includes outer shape data of the insole and hardness data of the insole with which position data is associated.

4. The information processing device according to any one of claims 1 to 3, wherein
the data on the bones of the foot includes position information of at least one type of bone selected from among metatarsals and a calcaneus.

5. The information processing device according to any one of claims 1 to 4, further comprising:
a foot information generator that generates the foot information, wherein
the foot information generator references a database in which types of bones, shapes of the bones and position information of the bones are associated with each other and stored, and generates, from the image data of the subject, data related to the bones of the foot of the subject and including information on the types of the bones, the shapes of the bones and the positions of the bones.

6. The information processing device according to any one of claims 1 to 5, further comprising:
an output unit that outputs the model data generated by the model data generator to the shaping device.

7. The information processing device according to any one of claims 1 to 6, further comprising:
a display device that displays the model data generated by the model data generator together with the foot information; and
a receiver that receives an operation of correcting the model data from a user who references the model data and the foot information displayed by the display device, wherein
the model data generator corrects the model data based on the operation received by the receiver.

8. The information processing device according to any one of claims 1 to 7, wherein
the image data is three-dimensional X-ray CT image data that is obtained by imaging and includes the foot of the subject.

9. The information processing device according to any one of claims 1 to 8, wherein
the model data generator generates the model data based on shoe shape information as well as the foot information.

10. The information processing device according to any one of claims 1 to 8, wherein
the image data of the subject is data obtained by imaging in a state in which the subject wears a shoe.

11. An information processing system comprising:
a foot information generator that generates, based on image data of a subject, foot information including outer shape data of a foot of the subject and data on bones of the foot of the subject;
a model data generator that generates, based on the foot information, model data to be used for a shaping device to shape an insole to be used by the subject;
a display device that displays the model data generated by the model data generator together with the foot information; and
a receiver that receives an operation of correcting the model data from a user who references the model data and the foot information displayed by the display device, wherein
the model data generator corrects the model data based on the operation received by the receiver.

12. An insole production system including at least an information processing device and a shaping device, comprising:
a foot information generator that generates, based on image data of a subject, foot information including outer shape data of a foot of the subject and data on bones of the foot of the subject;
a model data generator that generates, based on the foot information, model data to be used for the shaping device to shape an insole to be used by the subject; and
a shaping unit that shapes the insole based on the model data.

13. An insole production method comprising:
a foot information generation step of generating, based on image data of a subject, foot information including outer shape data of a foot of the subject and data on bones of the foot of the subject;
a model data generation step of generating, based on the foot information, model data to be used for a shaping device to shape an insole to be used by the subject; and
a shaping step of shaping the insole based on the model data.

14. An information processing method comprising:
a foot information generation step of generating, based on image data of a subject, foot information including outer shape data of a foot of the subject and data on bones of the foot of the subject; and
a model data generation step of generating, based on the foot information, model data to be used for a shaping device to shape an insole to be used by the subject.

15. A computer program that causes a computer to execute:
a foot information generation step of generating, based on image data of a subject, foot information including outer shape data of a foot of the subject and data on bones of the foot of the subject; and
a model data generation step of generating, based on the foot information, model data to be used for a shaping device to shape an insole to be used by the subject.
